(19) Europäisches Patentamt
European Patent Office
Office européen des brevets

(11) Numéro de publication: **0 130 882**
**B1**

(12) # FASCICULE DE BREVET EUROPEEN

(45) Date de publication du fascicule du brevet: **07.01.87**

(51) Int. Cl.⁴: **C 07 C 61/13**, A 61 K 31/19, A 61 K 31/16, C 07 C 69/753

(21) Numéro de dépôt: **84401276.5**

(22) Date de dépôt: **20.06.84**

(54) **Dérivés de l'acide bicyclo (3.2.1.) octane carboxylique, leur procédé de préparation et leur application thérapeutique.**

(30) Priorité: **22.06.83 FR 8310612**

(43) Date de publication de la demande: **09.01.85 Bulletin 85/2**

(45) Mention de la délivrance du brevet: **07.01.87 Bulletin 87/2**

(84) Etats contractants désignés: **AT BE CH DE FR GB IT LI LU NL SE**

(56) Documents cités: **FR - M - 4 983**

(73) Titulaire: **SANOFI, 40, Avenue George V, F-75008 Paris (FR)**

(72) Inventeur: **Boigegrain, Robert, 3 Rue Dumont d'Urville, F-31120 Portet/Garonne (FR)**
Inventeur: **Chenu, Jacques, 38 Chemin des Vivants, F-31600 Muret (FR)**
Inventeur: **Simiand, Jacques, 136 Chemin de Lacombe, F-31600 Muret (FR)**
Inventeur: **Vernieres, Jean-Claude, Rue Sabatier Garat, F-31600 Muret (FR)**

(74) Mandataire: **Bressand, Georges et al, c/o CABINET LAVOIX 2 Place d'Estienne d'Orves, F-75441 Paris Cedex 09 (FR)**

EP 0 130 882 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention est relative à de nouveaux dérivés de l'acide bicyclo (3.2.1.) octane carboxylique, à leur procédé de préparation et à leur application en médecine humaine et vétérinaire.

Les composés de l'invention répondent à la formule développée générale suivante:

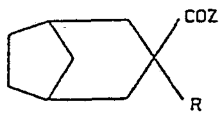

(I)

dans laquelle,

R représente un radical alcoyle inférieur droit ou ramifié,

Z représente le groupement OX dans lequel X est l'hydrogène ou un métal alcalin, ou

Z représente encore le groupe

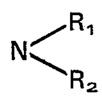

dans lequel $R^1$ et $R^2$ identiques ou différents sont l'hydrogène ou un radical alcoyle inférieur.

Par radical alcoyle inférieur, on entend une chaîne hydrocarbonée possédant de 1 à 4 atomes de carbone.

Ces composés peuvent exister sous forme de plusieurs stéréoisomères. L'invention concerne aussi bien chaque isomère que leur mélange.

Des composés possédant la même structure bicyclooctane carboxylique sont connus.

C'est ainsi que BUCHTA et BILLENSTEIN (Ann. Chem. 1966, 692, 42-52) ont préparé les composés pour lesquels Z et R ont les significations suivantes:

| R | Z |
|---|---|
| $COOC_2H_5$ | $OC_2H_5$ |
| $COOC_2H_5$ | OH |
| COOH | OH |
| H | $OC_2H_5$ |
| H | OH |

TAKEFUMI et OSAMU (Chem. Pharm. Bull, 1978, *26*, 8, 2589-2593) ont synthétisé les dérivés dans lesquels R étant H, Z représente $C_6H_5$ ou $-OC_6H_5$.

NELSON, Mc EVEN et LAWTON (J. ORG. CHEM. 1969, *34*, *5*, 1225-1229) ont préparé le dérivé suivant:

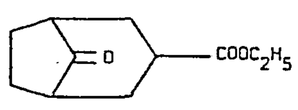

Néanmoins les dérivés décrits par BUCHTA et al. TAKEFUMI et Al et NELSON et al n'ont fait l'objet d'aucune étude Toxico-pharmacologique.

L'invention a également pour objet un procédé de préparation des composés de formule I précités, caractérisé en ce que l'oxo-8 bicyclo (3.2.1.) octane-carboxylate-3 de méthyle de formule

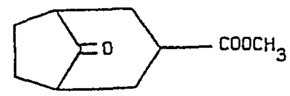

(II)

est transformé par réduction et hydrolyse en un acide de formule

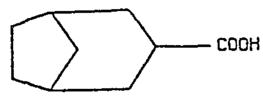

(III)

qui est mis à réagir avec une base forte pour former un composé organo-métallique intermédiaire de formule

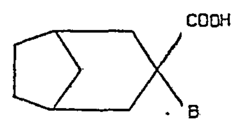

(IV)

dans laquelle $-B$ est un métal alcalin et qui, par action d'un halogénure d'alcoyle $R-X$ dans lequel R possède les significations décrites précédemment, est transformé en composé de formule I dans lequel Z représente OH. Par salification, estérification ou amidification on prépare, selon les méthodes classiques les composés de formule I dans lesquelles Z est différent de OH.

Le composé de formule II a été préparé selon la méthode décrite par NELSON et al (J. Org. chem. 1969 *34*, 1225-1229) utilisée pour préparer l'ester éthylique.

La réduction de ce composé, pour obtenir le composé de formule III, est réalisée par l'action d'un agent réducteur, en particulier la potasse et l'hydrazine. Ce composé de formule III a été décrit par E. BUCHTA et coll (Ann. chem. 1966, 692, 42, 52), mais ces derniers l'ont préparé par une voie différente.

La base forte utilisée pour obtenir le composé de formule IV peut être l'amidure de sodium, le diéthylamidure de lithium ou le diisopropylamidure de lithium.

A titre de variante, il peut être intéressant de transformer l'acide de formule III, en un dérivé tel que le nitrile ou un ester, avant de le faire réagir avec une base forte.

On obtient dans ce cas, après l'action du composé organo-métallique et de l'halogénure d'alcoyle, un dérivé de formule générale

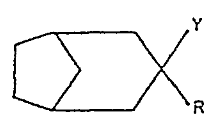

(V)

dans lequel Y représente une fonction nitrile ou ester, qu'on transformera ensuite en dérivé de formule I par des méthodes classiques.

Les exemples non limitatifs suivants illustrent l'invention.

*Exemple 1:*

Acide méthyl-3 bicyclo (3.2.1.) octane carboxylique-3 (dérivé N° 1).

a) Préparation de l'oxo-8 bicyclo (3.2.1.) octane-carboxylate-3 = de méthyle de formule II. A une solution de 53 g (0,386 mole de N-cyclopentényl pyrrolidine (fraîchement distillée) et 118 ml (0,85 mole) de triéthylamine anhydre dans 400 ml d'acétonitrile sec, on ajoute sous azote 100 g (0,386 mole) de BB dibromoisobutyrate de méthyle dans 250 ml d'acétonitrile sec en maintenant la température du mélange inférieure à 35° C. On porte ensuite le mélange réactionnel à 60° C pendant 15 heures, puis on introduit 28 ml d'acide acétique dans 150 ml d'eau, et on porte l'ensemble au reflux pendant 1 heure. L'acétonitrile est éliminé sous vide et on ajoute au mélange 1000 ml d'eau. On extrait plusieurs fois au chlorure de méthylène. Par distillation sous vide, on recueille 45 g (Rdt 64%) de produit dont le point d'ébullition (0,5 mm) est de 110°-114° C.

b) Préparation de l'acide bicyclo (3.2.1.) octane carboxylique-3 de formule III. On chauffe progressivement à 190°-200° C un mélange de 32 g (0,176 mole) du dérivé de formule II précédemment obtenu, 31 g de potasse, 21 ml d'hydrate d'hydrazine à 98% et 220 ml de diéthylène glycol.

Le chauffage du mélange à 200° C est prolongé pendant 4 heures. L'eau et le méthanol sont éliminés par distillation durant cette opération. A froid, on verse ensuite le mélange dans 1000 ml d'eau. La solution aqueuse est lavée à l'éther puis acidifiée par l'acide chlorhydrique concentré. L'acide de formule III est extrait au chlorure de méthylène: on recueille 23,4 g de cristaux incolores (RDT 87%) dont le point de fusion est de 94° C.

c) Préparation du bicyclo (3.2.1.) octane carboxylate-3 de méthyle. L'acide de formule III précédemment obtenu (23 g 0,15 mole) est chauffé au reflux pendant 20 heures, avec 75 ml de dichloroéthane, 22 ml de méthanol et 1 ml d'acide sulfurique concentré. A froid, on ajoute 300 ml d'éther, on lave à l'eau puis au bicarbonate de potassium 2N. Par distillation de la partie organique, on recueille 23 g (Rdt 91%) de bicyclo (3.2.1.) octane carboxylate-3 de méthyle, dont le point d'ébullition (0,1 mm) est de 64°-65° C.

d) Préparation du méthyl-3 bicyclo (3.2.1.) octane carboxylate-3 de méthyle. Sous atmosphère d'azote (sec) on introduit dans un tricol 110 ml (0,17 mole) de butyllithium en solution dans l'hexane (titre 1,56 M), 120 ml de tétrahydrofurane (THF) anhydre. La solution est refroidie à 0° C et on ajoute 17,2 g (0,17 mole) de di-isopropylamine (séchée) en solution dans 30 ml de T.H.F. On agite 15 minutes à 0° C puis on refroidit à 70°-80° C, et on ajoute lentement 23 g (0,137 mole) d'ester précédemment obtenu en solution dans 20 ml de T.H.F., puis on maintient le mélange à une température de 70°-80° C pendant 1 heure. On ajoute à la même température 24,2 g (0,17 mole) d'iodure de méthyle dans 25 ml de Hexaméthyl phosphore-triamide (H.M.P.T.). On laisse remonter la température du mélange entre 20° et 10° C et on abandonne pendant 4 heures.

Le mélange est hydrolysé à l'eau puis au benzène. La phase organique est lavée plusieurs fois par une solution d'acide chlorhydrique à 10% puis au bicarbonate de potassium 2N. On sépare la partie organique, par distillation on recueille 24 g (Rdt 92%) de méthyl-3 bicyclo (3.2.1.) octane carboxylate-3 de méthyle dont le point d'ébullition (0,05 mm) est de 45°-50° C.

e) Acide méthyl-3 bicyclo (3.2.1.) octane carboxylique-3 24 g de l'ester précédemment obtenu (0,133 mole) sont chauffés au reflux à 160° pendant 5 heures avec 14,5 g de potasse et 1000 ml de glycol.

A froid, on ajoute ensuite 200 ml d'eau et on extrait à l'éther. La phase aqueuse séparée est acidifiée. L'acide est extrait au benzène. On recueille ainsi 14 g de cristaux incolores (Rdt 62%) dont le point de fusion est de 109-110° C (Hexane).

*Exemple 2:*

Méthyl-3 bicyclo (3.2.1.) octane carboxylate-3 de sodium (dérivé N° 2)

16,8 g (0,01 mole) d'acide préparé précédemment dans l'exemple 1 sont ajoutés à une solution de méthylate de sodium préparé à partir de 2,3 g de sodium dans le méthanol. Le mélange est chauffé à 50° C pendant 15 minutes, puis le méthanol est évaporé sous vide. Les cristaux incolores de méthyl-3 bicyclo (3.2.1.) octane carboxylate-3 de sodium sont lavés à l'éther et séchés (Rdt 95%) F. 270° C.

*Exemple 3:*

Acide éthyl-3 bicyclo (3.2.1.) octane carboxylique-3 (dérivé N° 3)

En suivant le mode opératoire décrit dans l'exemple 1, on a préparé successivement à partir des réactifs convenables: l'éthyl-3 bicyclo (3.2.1.) octane carboxylate-3 de méthyle (Eb 4,5 mm = 91-92° C) puis l'acide éthyl-3 bicyclo (3.2.1.) octane carboxylique-3, dont le point de fusion est de 81-82° C.

*Exemple 4:*

L'acide isopropyl-3 bicyclo (3.2.1.) octane carboxylique-3 (dérivé N° 4)

En suivant le mode opératoire décrit dans l'exemple 1, on a préparé successivement à partir des réactifs appropriés: l'isopropyl-3 bicyclo (3.2.1.) octane carboxylate-3 de méthyle (Eb 5 mm = 94-96° C) puis l'acide isopropyl-3 bicyclo (3.2.1.) octane carboxylique-3 dont le point de fusion est de 72°-74° C.

*Exemple 5:*

Méthyl-3 bicyclo (3.2.1.) octane carboxamide-3 (dérivé N° 5)

8,7 g (0,0517 mole) d'acide préparé selon l'exemple 1 en solution dans 50 ml de dichloroéthane et 10 ml de chlorure de thionyle sont chauffés au reflux pendant 5 heures. On évapore à sec et

distille le chlorure d'acide sous vide (Eb 0,3 - 45-48° C). Il est ensuite additionné à une solution de dichloroéthane saturée en ammoniac.

On concentre à sec, lave l'amide à l'eau: on recueille 48 g de cristaux incolores (Rdt: 56%) dont le point de fusion est de 110°-111° C (éther isopropylique).

*Exemple 6:*

Ethyl-3 bicyclo (3.2.1.) octane carboxamide-3 (dérivé N° 6)

En suivant le mode opératoire décrit dans l'exemple 5, on a préparé à partir du dérivé N° 3, l'éthyl-3 bicyclo (3.2.1.) octane carboxamide-3, dont le point de fusion est de 101° C.

*Exemple 7:*

Isopropyl-3 bicyclo (3.2.1.) octane carboxamide-3 (dérivé N° 7)

En suivant le mode opératoire décrit dans l'exemple 5, on a préparé à partir du dérivé N° 4, l'isopropyl-3 bicyclo (3.2.1.) octane carboxamide-3, dont le point de fusion est de 142° C.

*Exemple 8:*

7 g (0,037 mole) de chlorure d'acide de l'acide méthyl-3 bicyclo (3.2.1.) octane carboxylique-3 préparé selon l'exemple 5 sont traités en solution dans 70 ml de dichloroéthane par 46,6 g de méthylamine et 5,2 ml de triéthylamine à une température de 0° C. Le mélange est abandonné pendant 4 heures. Après évaporation du solvant, on ajoute 50 ml d'eau et on extrait à l'éther l'amide que l'on purifie par passage sur une colonne de gel de silice: on obtient 5,1 g de cristaux incolores (Rdt: 76%) dont le point de fusion est de 91° C.

Les résultats des essais toxicologiques et pharmacologiques qui sont rapportés ci-après, ont mis en évidence les intéressantes propriétés des dérivés de l'invention, notamment une faible toxicité et une excellente tolérance, ainsi que d'importantes propriétés anticonvulsivante et antianoxique.

L'invention a donc encore pour objet un médicament ayant en particulier des activités anticonvulsivante et antianoxique caractérisé en ce qu'il contient, à titre de principe actif un dérivé de formule I. Tous les essais Toxico-pharmacologiques ont été pratiqués sur des souris mâles de souche CHARLES RIVER (CD1).

## ÉTUDE TOXICOLOGIQUE

Les composés de l'invention bénéficient d'une excellente tolérance et d'une faible toxicité.

Ainsi, la DL 50 déterminée par voie orale chez la souris par la méthode Finney (Probit analysis, University Press Cambridge, 1971) est de 3000 mg/kg pour le dérivé N° 5 et supérieure à 1000 mg/kg pour tous les composés de l'invention.

En outre, les essais effectués au cours des expérimentations de toxicité aiguë, chronique, subchronique, retardée et de tératogénicité n'ont pas permis de mettre en évidence une quelconque anomalie.

Dans le cadre de cette étude, on a déterminé l'activité sédative des composés de l'invention; on a utilisé dans ce but, le test du cylindre tournant (Boissier, J.R., Therapie 1958, 13, 1074).

Ce test est destiné à apprécier l'aptitude des animaux à coordonner leurs mouvements. Il met en œuvre un cylindre de bois, d'un diamètre de 4,8 cm, qui tourne à la vitesse de 4 tours/min. Le test est pratiqué sur des souris d'un poids de 22 g environ. Les produits sont administrés par voie orale à différents lots de 10 animaux. Ces derniers sont placés sur l'appareil 15 minutes après l'administration du produit. On note le pourcentage de souris qui perdent le réflexe d'équilibration, c'est-à-dire qui deviennent incapables de se maintenir sur le cylindre pendant une durée de 2 minutes.

Par la méthode de Finney, on calcule une dose neurotoxique 50 (DNT 50) qui exprime donc le niveau moyen des premières atteintes neuromusculaires sous l'influence d'un produit étudié.

Ainsi déterminées les DNT 50 sont:

Composé N° 2 = 600 mg/kg
5 = 365 mg/kg
6 = 400 mg/kg
7 = 230 mg/kg
8 = 500 mg/kg

## ÉTUDE PHARMACOLOGIQUE

I - Activité anticonvulsivante

*1) Crise pentétrazolique*

Ce test a été effectué sur des lots de 10 souris à jeun depuis la veille et mises en cages individuelles, selon la méthode Cheymol (J., Acta. Pharmacol., 1950, 2, 1-55).

Les traitements sont faits par voie orale 15 minutes avant l'injection par voie intrapéritonéale d'une solution de pentétrazole (125 mg/kg). On note dans chaque lot le pourcentage de protection contre les crises toniques. Les doses efficaces 50 (DE 50) calculées par la méthode de Finney, exprimées en mg/kg de poids corporels, sont les suivantes:

Composé N° 2 = 90
5 = 53
6 = 75
7 = 120
8 = 115

A titre de comparaison, la DE 50 du valproate de sodium agent anticonvulsivant très actif, est de 200 mg/kg.

*2) Crise électrique supramaximale*

Cette expérimentation a été effectuée selon le test de Swinyard et al. (Am. J. Pharmacol, Ther., 1952, 168-175). Les traitements ont été administrés per os à des lots de 10 souris 30 min, avant l'électrochoc supramaximal (courant alternatif de 50 HZ, 60 volts pendant 0,4 sec.) délivré par l'intermédiaire d'électrodes intra-auriculaires. On note le nombre d'animaux dans chaque lot qui ne présentent pas d'hyperextension tonique lors de la stimulation électrique transcrânienne, et on détermine la DE 50 exprimée en mg/kg.

Composé N° 2 = 400
5 = 210
6 = 210
7 = 260
8 = 310

*3) Crise à la bicuculline*

Ce test a été effectué selon la méthode de Pérez de la Mora et Tapia (Biochem. Pharmacol., 1973, 19, 303-310). Les traitements ont été administrés par voie orale à des lots de 10 souris, 30 minutes avant l'injection de bicuculline (0,65 mg/kg) par la voie intraveineuse.

L'apparition des convulsions toniques a été notée pendant les 30 min qui ont suivi l'injection de bicuculline. Les DE 50 en mg/kg ainsi déterminées sont:

Composé N° 5 = 68
6 = 61
7 = 145
8 = 75

*4) Crise à l'acide B-mercaptopropionique*

L'acide B-mercaptopropionique est un inhibiteur de la glutamate-décarboxylase (GAD), enzyme qui catalyse la transformation du glutamate en acide Y-aminobutyrique (GABA). Son action se traduit donc par un abaissement du taux de GABA et la survenue de crises généralisées tonicocloniques 3 à 4 minutes après son administration. L'acide B-mercaptopropionique a été administré aux souris par voie I.P. à la dose de 60 mg/kg. Le traitement pour les composés à tester est administré par la voie orale 15 minutes avant l'injection d'acide B-mercaptopionique. Les DE 50 exprimées en mg/kg, ainsi déterminées sont:

Composé N° 2 = 90
5 = 30
6 = 60
7 = 120
8 = 70

*II - Activité antianoxique*

Cette activité a été étudiée par la méthode de l'anoxie par confinement.

Des souris (20 par lot) sont placées dans une enceinte (bocal en verre) normobare, normoxique, hermétiquement close de 200 ml. Tous les traitements sont administrés par voie I.P. immédiatement avant l'introduction dans l'enceinte. L'animal est considéré comme mort au moment de l'arrêt respiratoire.

Les bocaux choisis étant très petits, l'activité motrice des animaux est extrêmement faible, ce qui élimine en principe la possibilité d'un résultat positif par effet sédatif. On note dans chaque lot le temps de survie des animaux.

Ce temps est augmenté de 15% pour le dérivé N° 2 et de 25% pour le dérivé N° 5.

Ces expérimentations toxicologique et pharmacologique ont permis de constater que l'indice thérapeutique des composés de l'invention est très favorable puisque, comme on a pu le constater pour tous les composés, les doses toxiques et neurotoxiques sont très éloignées des DE 50.

En outre, les composés de l'invention sont doués d'intéressantes activités anticonvulsivante et antianoxique qui les rendent très précieux en thérapeutique.

Le médicament de l'invention peut-être présenté pour l'administration orale sous forme de comprimés, comprimés dragéifiés, capsules, gouttes, granulés ou sirop. Il peut être aussi présenté pour l'administration rectale sous forme de suppositoires et pour l'administration parentérale sous forme de soluté injectable.

Chaque dose unitaire contient avantageusement de 0,050 g à 0,500 g de principe actif, les doses administrables journellement pouvant varier de 0,050 g à 3,00 g de principe actif en fonction de l'âge du patient et de la sévérité de l'affection traitée.

On donnera ci-après, à titre d'exemples non limitatifs, quelques formulations pharmaceutiques du médicament de l'invention:

1. Comprimés
   Dérivé N° 2: 0,200 g
   Excipient: Silicate de calcium, polyvinyl pyrrolidone, stéarate de magnésium, talc, amidon de maïs, oxyde de titane, acétyphtalate de cellulose.
2. Comprimés dragéifiés
   Dérivé N° 5: 0,100 g
   Excipient: Levilite amidon, gélatine, stéarate de magnésium, gomme laque, talc, gomme arabique, saccharose, oxyde de titane.
3. Gélules
   Dérivé N° 6: 0,150 g
   Excipient: Talc, stéarate de magnésium.
4. Sirop
   Dérivé N° 5: 1,00 g
   Excipient aromatisé qsp 100 ml.
5. Soluté injectable
   Dérivé N° 7: 0,150 g
   Solvant isotonique qsp 5 ml.
6. Suppositoire
   Dérivé N° 8: 0,100 g
   Glycérides semi-synthétiques qsp 1 suppositoire.

Par son spectre d'activité neuropsychotrope, doué d'importants effets anticonvulsivant et antianoxique, respectant la vigilance, le médicament de l'invention est utilisé avec profit, aussi bien chez l'adulte que chez l'enfant, dans le traitement des troubles thymiques aigus ou chroniques tels que cyclothymie, psychose maniaco-dépressive, accès maniaques, états dépressifs, mélancolie à rechutes, dans les manifestations psychotiques diverses, dans les troubles du caractère et du comportement, dans les troubles du sommeil et dans toutes les formes d'épilepsie, ainsi que dans les troubles liés au vieillissement cérébral.

**Revendications** pour les Etats contractants BE, CH, DE, FR, GB, IT, LI, LU, NL, SE

1. Dérivés de l'acide bicyclo (3.2.1.) octane

carboxylique-3 répondant à sa formule générale suivante:

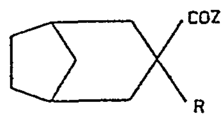

(I)

dans laquelle,

R représente un radical alcoyle inférieur droit ou ramifié

Z représente le groupement OX dans lequel X est l'hydrogène ou un métal alcalin, ou

Z représente encore le groupe

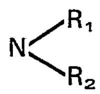

dans lequel $R_1$ et $R_2$ identiques ou différents, sont l'hydrogène ou un radical alcoyle inférieur.

2. Procédé de préparation des dérivés de formule I, caractérisé en ce que l'oxo-8 bicyclo (3.2.1.) octane-carboxylate-3 de méthyle de formule

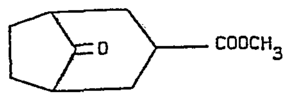

(II)

est transformé en un acide de formule

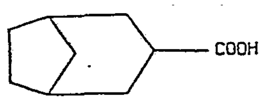

(III)

qui est mis à réagir avec une base forte pour former un composé organo-métallique intermédiaire de formule

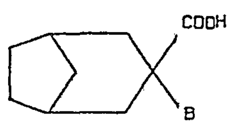

(IV)

dans laquelle B est un métal alcalin, et qui par action d'un halogénure d'alcoyle R−X dans lequel R possède les significations décrites précédemment, est transformé en composé de formule I dans lequel Z représente OH, et on prépare selon les méthodes classiques les composés de formule I dans lesquels Z est différent de OH.

3. Procédé selon la revendication 2, caractérisé en ce que le dérivé de formule III est préparé en faisant réagir l'oxo-8 bicyclo (3.2.1.) octane-carboxylate-3 de méthyle avec de la potasse et de l'hydrazine.

4. Procédé selon la revendication 2, caractérisé en ce que selon une variante, l'acide de formule III peut être remplacé par le nitrile ou un ester obtenant ainsi un composé de formule V qui est ensuite transformé en composé de formule I par des méthodes classiques.

5. Médicament présentant des activités anticonvulsivante et antianoxique, caractérisé en ce qu'il contient, à titre de principe actif un composé selon la revendication 1.

6. Médicament selon la revendication 5, caractérisé en ce qu'il est présenté sous une forme appropriée à l'administration orale, parentérale ou rectale, le principe actif étant associé à un véhicule thérapeutiquement administrable.

7. Médicament selon la revendication 6, caractérisé en ce qu'il est présenté sous forme de doses unitaires contenant chacune de 0,050 g à 0,500 g de principe actif.

**Revendications** pour l'Etat contractant: AT

1. Procédé de préparation de dérivés de l'acide bicyclo (3.2.1.) octane carboxylique-3 répondant à sa formule générale suivante:

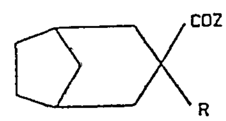

(I)

dans laquelle,

R représente un radical alcoyle inférieur droit ou ramifié

Z représente le groupement OX dans lequel X est l'hydrogène ou un métal alcalin, ou

Z représente encore le groupe

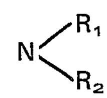

dans lequel $R_1$ et $R_2$ identiques ou différents, sont l'hydrogène ou un radical alcoyle inférieur, caractérisé en ce que l'oxo-8 bicyclo (3.2.1.) octane-carboxylate-3 de méthyle de formule

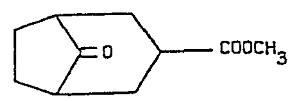

(II)

est transformé en un acide de formule

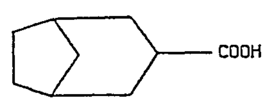

(III)

qui est mis à réagir avec une base forte pour former un composé organo-métallique intermédiaire de formule

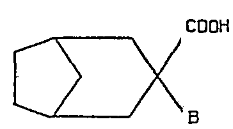

(IV)

dans laquelle B est un métal alcalin, et qui par action d'un halogénure d'alcoyle R−X dans lequel R possède les significations décrites précédemment, est transformé en composé de formule I dans lequel Z représente OH, et on prépare selon les méthodes classiques les composés de formule I dans lesquels Z est différent de OH.

2. Procédé selon la revendication 1, caractérisé en ce que le dérivé de formule III est préparé en faisant réagir l'oxo-8 bicyclo (3.2.1.) octane-carboxylate-3 de méthyle avec de la potasse et de l'hydrazine.

3. Procédé selon la revendication 1, caractérisé en ce que selon une variante, l'acide de formule III

peut être remplacé par le nitrile ou un ester obtenant ainsi un composé de formule V qui est ensuite transformé en composé de formule I par des méthodes classiques.

4. Procédé de préparation d'un médicament présentant des activités anticonvulsivante et antianoxique, caractérisé en ce qu'on présente sous une forme appropriée à l'administration orale, parentérale ou rectale un composé selon la revendication 1.

**Patentansprüche** für die Vertragsstaaten BE, CH, DE, FR, GB, IT, LI, LU, NL, SE

1. Derivate der Bicyclo (3.2.1.)-3-oktancarbonsäure der allgemeinen Formel:

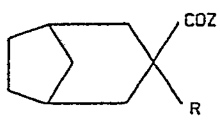
(I)

in der R eine niedere, geradkettige oder verzweigte Alkylgruppe,

Z die OX-Gruppe, in der X Wasserstoff oder ein Alkalimetall ist, oder

Z noch die Gruppe

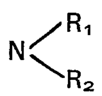

in der $R_1$ und $R_2$, verschieden oder gleich, Wasserstoff oder eine niedere Alkylgruppe sind, bedeuten.

2. Verfahren zur Herstellung der Derivate gemäss Formel I, dadurch gekennzeichnet, dass man 8-Oxo-bicyclo (3.2.1.)-3-oktanmethylcarboxylat der Formel

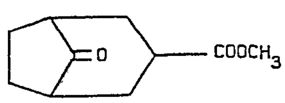
(II)

in eine Säure der Formel

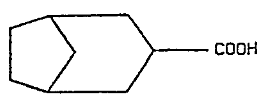
(III)

umwandelt, die mit einer starken Base zur Bildung einer metallorganischen Zwischenverbindung der Formel

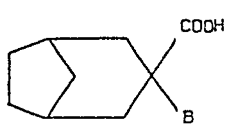
(IV)

umgesetzt wird, wobei B ein Alkalimetall ist, und die durch Einwirkung eines Alkylhalogenids R–X, in der R die vorbeschriebenen Bedeutungen besitzt, in eine Verbindung der Formel I umgewandelt wird, in der Z OH ist, und dass in üblicher Weise die Verbindungen gemäss Formel I hergestellt werden, in denen Z verschieden von OH ist.

3. Verfahren nach Anspruch 2, dadurch gekennzeichnet, dass das Derivat der Formel III hergestellt wird, indem man 8-Oxo-bicyclo (3.2.1.)-3-oktanmethylcarboxylat mit Kaliumcarbonat und Hydrazin umsetzt.

4. Verfahren nach Anspruch 2, dadurch gekennzeichnet, dass man gemäss einer Abwandlung die Säure gemäss Formel III durch das Nitril oder einen Ester ersetzt, wobei man ebenfalls eine Verbindung der Formel V erhält, die dann in üblicher Weise in eine Verbindung der Formel I umgewandelt wird.

5. Arzneimittel mit Antikonvulsions- und Antianoxiewirkung, dadurch gekennzeichnet, dass es als aktiven Wirkstoff eine Verbindung gemäss Formel I enthält.

6. Arzneimittel gemäss Anspruch 5, dadurch gekennzeichnet, dass es in geeigneter Form zur oralen, parenteralen oder rectalen Verabreichung vorliegt, wobei der aktive Wirkstoff mit einem therapeutisch verabreichbaren Träger zusammengebracht ist.

7. Arzneimittel gemäss Anspruch 6, dadurch gekennzeichnet, dass es in Form von Einheitsdosen mit einem Gehalt von jeweils 0,050 g bis 0,500 g aktivem Wirkstoff vorliegt.

**Patentansprüche** für den Vertragsstaat: AT

1. Verfahren zur Herstellung von Derivaten der Bicyclo (3.2.1.)-3-oktancarbonsäure der allgemeinen Formel:

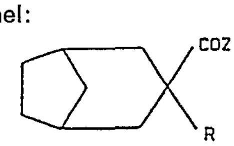
(I)

in der R eine niedere, geradkettige oder verzweigte Alkylgruppe,

Z die OX-Gruppe, in der X Wasserstoff oder ein Alkalimetall ist, oder

Z noch die Gruppe

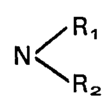

in der $R_1$ und $R_2$, verschieden oder gleich, Wasserstoff oder eine niedere Alkylgruppe sind, bedeuten, dadurch gekennzeichnet, dass man 8-Oxo-bicyclo (3.2.1.)-3-oktanmethylcarboxylat der Formel

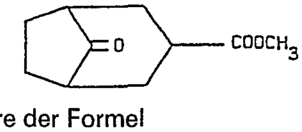
(II)

in eine Säure der Formel

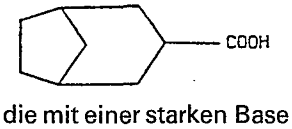
(III)

umwandelt, die mit einer starken Base zur Bildung einer metallorganischen Zwischenverbindung der Formel

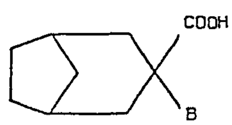
(IV)

umgesetzt wird, wobei B ein Alkalimetall ist, und die durch Einwirkung eines Alkylhalogenids R−X, in der R die vorbeschriebenen Bedeutungen besitzt, in eine Verbindung der Formel I umgewandelt wird, in der Z OH ist, und dass in üblicher Weise die Verbindungen gemäss Formel I hergestellt werden, in denen Z verschieden von OH ist.

2. Verfahren nach Anspruch 1, dadurch gekennzeichnet, dass das Derivat der Formel III hergestellt wird, indem man 8-Oxo-bicyclo (3.2.1.)-3-oktanmethylcarboxylat mit Kaliumcarbonat und Hydrazin umsetzt.

3. Verfahren nach Anspruch 1, dadurch gekennzeichnet, dass man gemäss einer Abwandlung die Säure gemäss Formel III durch das Nitril oder einen Ester ersetzt, wobei man ebenfalls eine Verbindung der Formel V erhält, die dann in üblicher Weise in eine Verbindung der Formel I umgewandelt wird.

4. Verfahren zur Herstellung eines Arzneimittels mit Antikonvulsions- und Antianoxiewirkung, dadurch gekennzeichnet, dass man in zur oralen, parenteralen oder rectalen Verabreichung geeigneter Form eine Verbindung gemäss Anspruch 1 zur Verfügung stellt.

**Claims** for the Contracting States BE, CH, DE, FR, GB, IT, LI, LU, NL, SE

1. Bicyclo (3.2.1.) octane-3-carboxylic acid derivatives, having the following general formula:

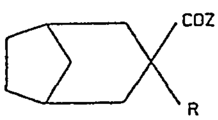 (I)

in which:

R represents a straight- or branched-chain lower alkyl radical

Z represents a group OX in which X is hydrogen or an alkaline metal, or

Z may also represent a group

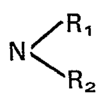

in which $R_1$ and $R_2$, which may be the same or different, are hydrogen or a lower alkyl radical.

2. Process for the preparation of derivatives of the formula (I) as defined in claim 1, comprising converting methyl 8-oxo-bicyclo (3.2.1.) octane-3-carboxylate having the formula:

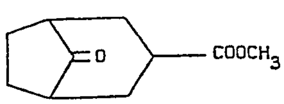 (II)

to an acid of the formula

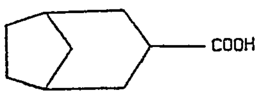 (III)

reacting the acid of the formula (III) with a strong base to give an intermediate organometallic compound having the formula:

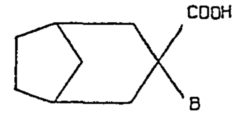 (IV)

in which B is an alkaline metal and, by action of an alkyl halide R−X in which R has the aforedisclosed meanings, converting coumpound (IV) to a compound of the formula (I) in which Z is OH, and preparing according to conventional methods the compounds of the formula (I) in which Z is other than OH.

3. Process as claimed in claim 2, wherein the derivative of the formula (III) is prepared by reacting methyl 8-oxo-bicyclo (3.2.1.) octane-3-carboxylate with potassium hydroxide and hydrazine.

4. Process as claimed in claim 2, wherein, according to a modification, the acid of the formula (III) may be substituted with the nitrile or an ester, thus giving a compound of the formula (V) which is then converted to a compound of the formula (I) by conventional means.

5. Medicament having in particular anticonvulsant and antianoxemic activities, comprising as active ingredient a compound as claimed in claim 1.

6. Medicament as claimed in claim 5, formulated in a form suitable for oral, parenteral or rectal administration, the active ingredient being combined with a therapeutically administrable vehicle.

7. Medicament as claimed in claim 6, in unit dosage form, each unit dose containing 0,050-0,500 g active ingredient.

**Claims** for the Contracting State: AT

1. Process for preparing Bicyclo (3.2.1.) octane-3-carboxylic acid derivatives, having the following general formula:

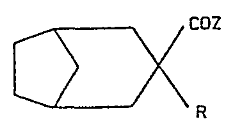 (I)

in which:

R represents a straight- or branched-chain lower alkyl radical

Z represents a group OX in which X is hydrogen or an alkaline metal, or

Z may also represent a group

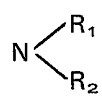

in which $R_1$ and $R_2$, which may be the same or different, are hydrogen or a lower alkyl radical, comprising converting methyl 8-oxo-bicyclo (3.2.1.) octane-3-carboxylate having the formula:

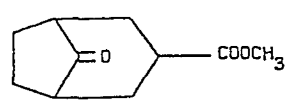 (II)

to an acid of the formula

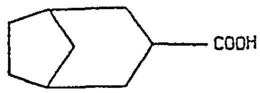

(III)

reacting the acid of the formula (III) with a strong base to give an intermediate organometallic compound having the formula

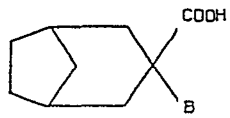

(IV)

in which B is an alkaline metal and, by action of an alkyl halide R−X in which R has the aforedisclosed meanings, converting compound (IV) to a compound of the formula (I) in which Z is OH, and preparing according to conventional methods the compounds of the formula (I) in which Z is other than OH.

2. Process as claimed in claim 1, wherein the derivative of the formula (III) is prepared by reacting methyl 8-oxo-bicyclo (3.2.1.) octane-3-carboxylate with potassium hydroxide and hydrazine.

3. Process as claimed in claim 1, wherein, according to a modification, the acid of the formula (III) may be substituted with the nitrile or an ester, thus giving a compound of the formula (V) which is then converted to a compound of the formula (I) by conventional means.

4. Process for preparing a medicament having in particular anticonvulsant and antianoxemic activities, wherein a compound as claimed in claim 1 is formulated in a form suitable for oral, parenteral or rectal administration.